(19) Europäisches Patentamt
European Patent Office
Office européen des brevets

(11) **EP 3 511 421 A1**

(12) **EUROPEAN PATENT APPLICATION**
published in accordance with Art. 153(4) EPC

(43) Date of publication:
**17.07.2019 Bulletin 2019/29**

(51) Int Cl.:
***C12Q 1/68*** *(2018.01)*

(21) Application number: **17849078.5**

(22) Date of filing: **06.09.2017**

(86) International application number:
**PCT/KR2017/009765**

(87) International publication number:
**WO 2018/048194 (15.03.2018 Gazette 2018/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **07.09.2016 KR 20160115024**
**04.09.2017 KR 20170112474**

(71) Applicants:
• **University Of Ulsan**
**Foundation For Industry Cooperation**
**Ulsan 44610 (KR)**
• **The Asan Foundation**
**Seoul 05505 (KR)**

(72) Inventors:
• **SHIN, Yong**
**Seoul 05505 (KR)**
• **KIM, Yong Sub**
**Seoul 05505 (KR)**

(74) Representative: **Roos, Peter**
**c/o Roospatent**
**Noldinstrasse 7**
**81545 München (DE)**

(54) **COMPOSITION AND METHOD FOR IMPROVING SENSITIVITY AND SPECIFICITY OF DETECTION OF NUCLEIC ACIDS USING DCAS9 PROTEIN AND GRNA BINDING TO TARGET NUCLEIC ACID SEQUENCE**

(57) The present invention relates to a composition and a method for improving sensitivity and specificity of detection of nucleic acids using a dCas9 protein. On the basis of a functional difference between Cas9 and dCas9, using dCas9 and gRNA, which binds to a target nucleic acid sequence, in amplification of nucleic acids including DNAs, RNAs, and the like increases efficiency in amplification of nucleic acids and thereby can ultimately improve sensitivity and specificity of a target diagnosis.

In particular, using dCas9 and gRNA thereof in a biosensor almost doubles the efficiency in amplification of nucleic acids, increases four times the difference between a non-target and a target, and prevents nonspecific amplification of a non-target, thereby greatly increasing sensitivity and specificity. Thus, the composition can be very usefully utilized for improving sensitivity and specificity of detection of nucleic acids.

FIG. 1

EP 3 511 421 A1

**Description**

**[Technical Field]**

The present invention relates to a composition and a method of improving the sensitivity and specificity of nucleic acid detection using dCas9 protein and gRNA binding to a target nucleic acid sequence.

**[Background Art]**

Methods for labeling and detecting nucleic acids which are difficult to detect in their natural state have been applied to various fields of molecular biology and cell biology. In order to detect signals in Southern blotting, Northern blotting, in situ hybridization and nucleic acid microarray using a specific hybridization reaction, nucleic acid to which labeling substance is attached has been widely used. A method of amplifying DNA and labeling DNA at the same time using a labeled monomer (labeled dNTP) or a labeled primer in a polymerase chain reaction (PCR) is known. The DNA thus labeled can be detected by a microarray.

The method of labeling the nucleic acid at the same time as the PCR has an advantage in that a separate step for labeling is not required, but when the monomer labeled with a fluorescent dye or the like is used, it has lower PCR efficiency than the case of using the unlabeled monomer. In addition, since RNA cannot be amplified by the PCR method, a step of preparing cDNA by reverse transcription is required so as to detect the RNA by the PCR-labeled method, and especially, when the length is short like microRNA (miRNA), it has a problem that cDNA production is cumbersome. Accordingly, there is an urgent need to develop a nucleic acid detection technique having improved sensitivity and specificity.

The above-described methods are easy to detect a target nucleic acid when a large amount of the detected nucleic acid is retained. Although it is still widely used today, when a small amount of target nucleic acid is present, it is very difficult to detect it (low sensitivity), and frequently, it cannot detect only a specific target and erroneously it detect a non-specific target, due to other inhibitors (low specificity).

Sensitivity and specificity are very low due to more inhibitors when directly detecting in body fluids of patients such as blood and urine, etc. Newly launched viruses or bacteria nowadays worldwide require early diagnosis and treatment, so methods that can detect it quickly and accurately are needed. Because in new variant diseases, target factors are present in very small amounts, development of a more sensitive and specific method is urgent.

In addition to this, it is necessary to apply the process having improved sensitivity and specificity to method of identifying mutations of biomarkers that cause cancer. It is important to develop technologies that can increase sensitivity and specificity by combining conventional technologies as much as the development of new technologies with high sensitivity and specificity.

**[Disclosure]**

**[Technical Problem]**

An object of the present invention is to provide a composition for improving the sensitivity and specificity of nucleic acid detection, comprising dCas9 (dead Cas, nuclease-inactive Cas9) protein and gRNA (guide RNA) binding to a target nucleic acid sequence, as an active ingredient and a method of improving the sensitivity and specificity of nucleic acid detection using the same.

**[Technical Solution]**

In order to accomplish the above object, the present invention provides a composition for improving sensitivity and specificity of nucleic acid detection, comprising: protein binding to a target nucleic acid sequence; or a complex of the protein and a gRNA (guide RNA) binding thereto, as an active ingredient.

Also, the present invention provides a composition for improving sensitivity and specificity of nucleic acid detection, comprising dCas9 (dead Cas, nuclease-inactive Cas9) protein and gRNA (guide RNA) binding to a target nucleic acid sequence, as an active ingredient.

In addition the present invention provides a kit for improving sensitivity and specificity of nucleic acid detection, comprising dCas9 (dead Cas, nuclease-inactive Cas9) protein and gRNA (guide RNA) binding to a target nucleic acid sequence, as an active ingredient.

Furthermore, the present invention provides a method of improving the sensitivity and specificity of nucleic acid detection, comprising amplifying a target nucleic acid by adding a dCas9 protein and a gRNA (guide RNA) binding to a target nucleic acid sequence to a sample containing the target nucleic acid; and detecting an amplified target nucleic acid amplification product.

**[Advantageous Effects]**

The present invention relates to a composition and a method for improving the sensitivity and specificity of nucleic acid detection using a dCas9 protein and a gRNA binding to a target nucleic acid sequence. Based on the functional difference between Cas9 having binding and cleavage function and dCas9 having only binding function and inactive cleavage function, when dCas9 protein and gRNA binding to a target nucleic acid sequence are used for nucleic acid amplification such as DNA and RNA, the efficiency of nucleic acid amplification is increased thereby ultimately achieving excellent sensitivity and specificity of the target diagnosis.

In addition, when the composition for improving the sensitivity and specificity of nucleic acid detection using dCas9 protein and gRNA binding to a target nucleic acid sequence according to the present invention is applied to a biosensor, the amplification efficiency is almost twice as high as that of the prior one and the difference between non-target and target increases by at least four times. In particular, since non-specific amplification of non-target is prevented, sensitivity and specificity are improved remarkably and therefore, the present invention can be utilized for nucleic acid detection with improved sensitivity and specificity.

**[Description of Drawings]**

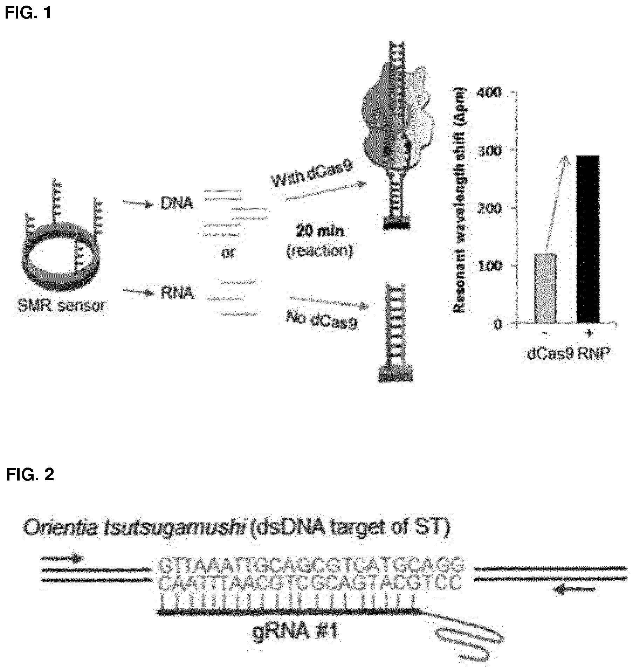

FIG. 1 shows a schematic diagram of a CRISPR-mediated biosensor according to the present invention (SMR sensor: silicon microring resonator sensor, with dCas9: using dCas9, No dCas9: dCas9 unused).

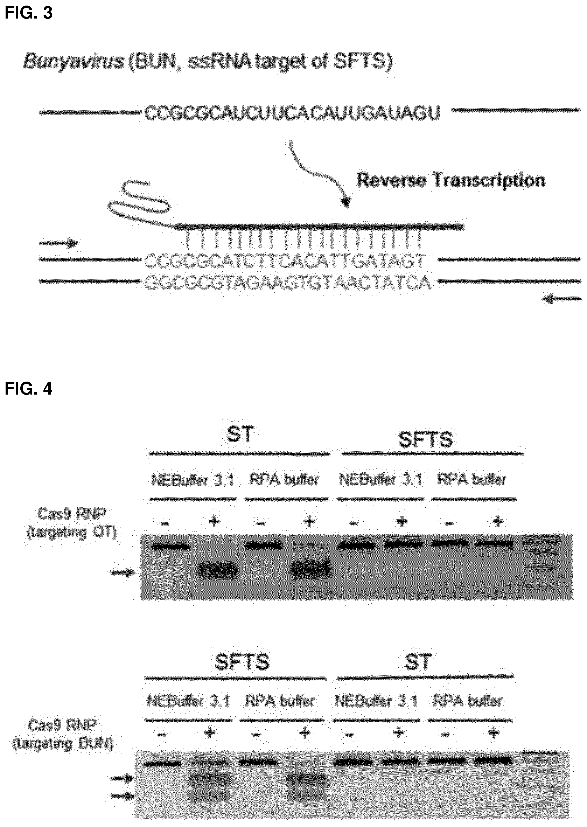

FIG. 2 and FIG. 3 illustrate schematic diagrams of gRNAs design prepared for detection of Orientia tsutsugamushi (OT), a causative organism of scrub typhus (ST) and Bunyavirus, a causative organism of severe fever with thrombocytopenia syndrome (SFTS) (DsDNA: double stranded DNA, ssRNA: single stranded RNA, target region highlighted in blue and PAM sequence shown in red).

FIG. 4 illustrates in vitro cleavage analysis for confirming gRNA activity under buffer conditions indicating that only when the gRNA is matched to the target PCR product, Cas9 RNP cleaves the PRC product in both RPA buffer and NEBuffer 3.1 conditions (ST: scrub typhus, SFTS: severe fever with thrombocytopenia syndrome).

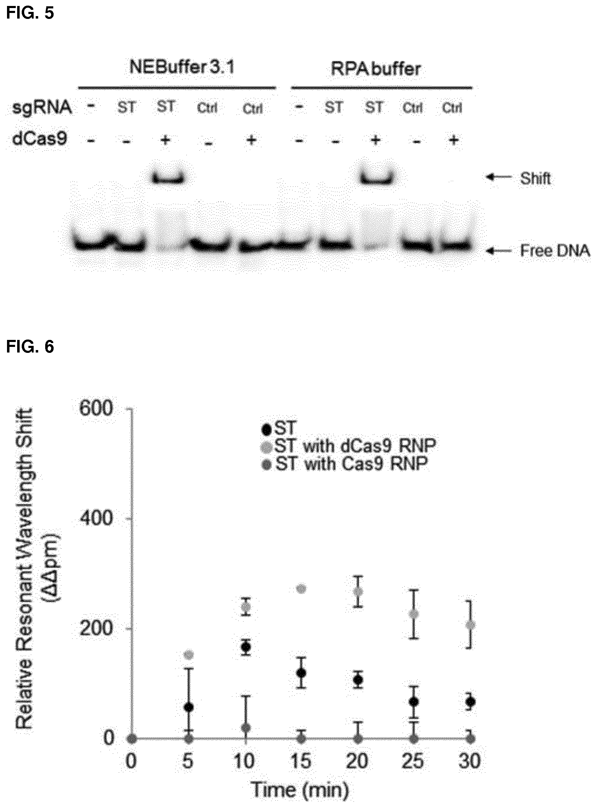

FIG. 5 shows the results of EMSA analysis using dCas9 RNP and 5'-biotinylated DNA duplexes, indicating that the target DNA duplexes were not cleaved but transferred by matched gRNAs in both RPA buffer and NEBuffer 3.1 conditions (ST: scrub typhus, Ctrl: control without target nucleic acid, sgRNA: single guide RNA).

FIG. 6 shows the result of ST-DNA detection within 30 min of the CRISPR-mediated biosensor (ST: SMR biosensor alone, ST with dCas9 RNP: dCas9-treated SMR biosensor, ST with Cas9 RNP: Cas9-treated SMR biosensor) (ST: scrub typhus, ST with dCas9 RNP: ST detection using dCas9, ST with Cas9 RNP: No dCas9: dCas9-unused ST detection).

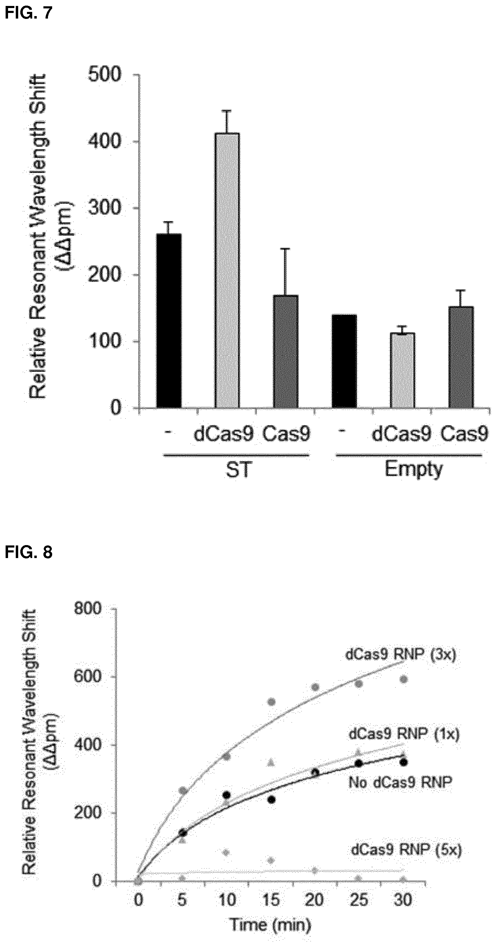

FIG. 7 shows the resonance wavelength shift result for ST detection within 15 minutes of a CRISPR-mediated biosensor (SMR biosensor alone: black, with dCas9 RNP: light gray, with Cas9 RNP: dark gray) (ST: scrub typhus, Empty: control group without target nucleic acid).

FIG. 8 shows the relative resonance wavelength shift result of a CRISPR-mediated biosensor for 30 minutes [ST: scrub typhus, dCas9 RNP; ST without dCas9 RNP, ST with 1x dCas9 RNP (100 ng dCas9 + 75 ng gRNA), ST with 3x dCas9 RNP (300 ng dCas9 + 225 ng gRNA), and ST with 5x dCas9 RNP (500 ng dCas9 + 375 ng gRNA)].

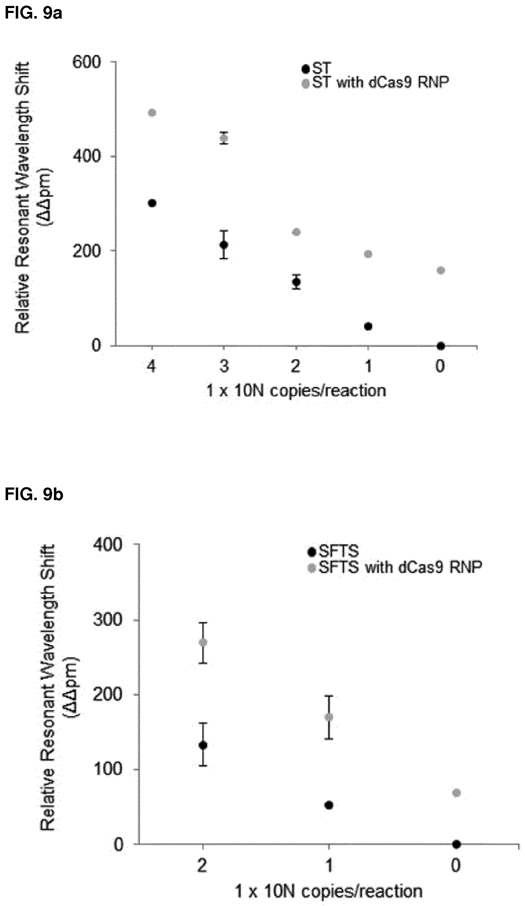

FIG. 9a shows the detection limit of dsDNA of ST according to the CRISPR-mediated biosensor (0.54 aM or less, grey), which is more sensitive than the SMR biosensor alone (black) (ST: scrub typhus, ST with dCas9 RNP: ST detection using dCas9 RNP).

FIG. 9b shows the detection limit of RNA of SFTS according to the CRISPR-mediated biosensor (0.63 aM or less, grey), which is more sensitive than the SMR biosensor alone (black) (SFTS: fever with thrombocytopenia syndrome, SFTS with dCas9 RNP: SFTS detection using dCas9 RNP),

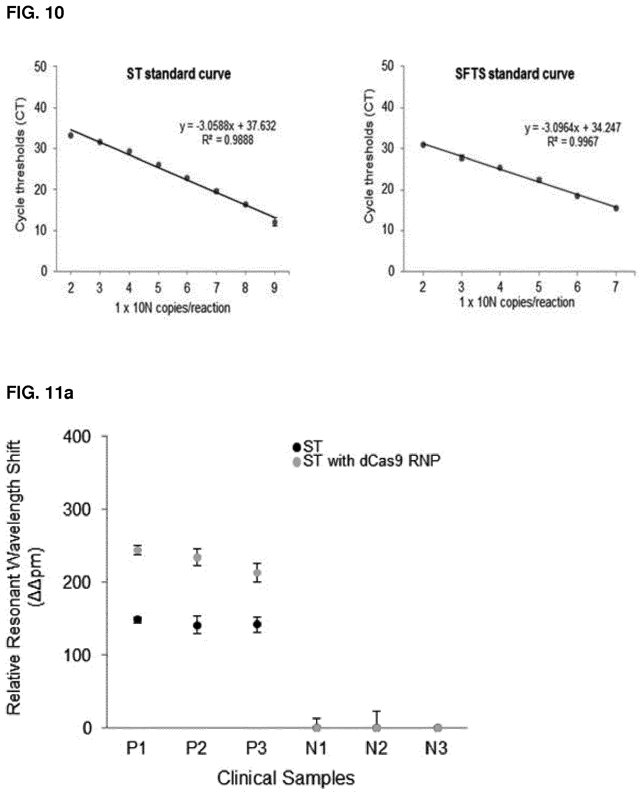

FIG. 10 shows the detection limit of real-time PCR for DNA and the detection limit of real-time RT-PCR for RNA, indicating that (a) real-time PCR showed a linear correlation between the concentration of the target DNA and the Ct value of the fluorescence signal, and a low concentration (<100 copies/ml) of target DNA was not detected (over 40 Ct value) and (b) real-time RT-PCR showed a linear correlation between the concentration of the target RNA and the Ct value of the fluorescence signal, and a low concentration (<100 copies/ml) of target RNA was not detected (over 40 Ct value) (ST standard curve, SFTS standard curve).

FIG. 11a shows high sensitive and specific detection of ST (gray) from a clinical sample according to a CRISPR-mediated biosensor, which was detected more sensitively and specifically than the SMR biosensor alone (black) (P1-3: positive as serum of ST patient, N1-3: negative as serum of SFTS patient).

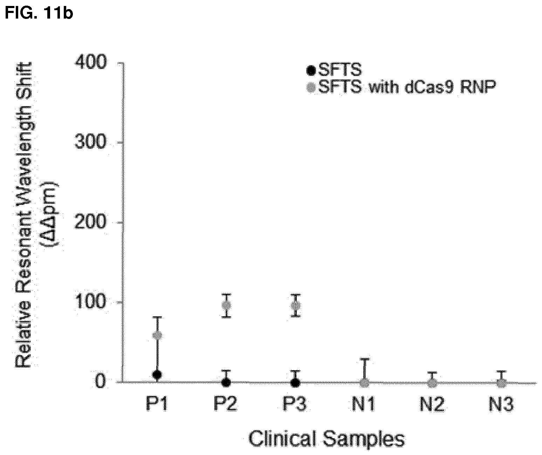

FIG. 11b high sensitive and specific detection of SFTS-RNA (gray) from a clinical sample according to a CRISPR-mediated biosensor, which was detected more sensitively and specifically than the SMR biosensor alone (black) (P1-3: positive as serum of SFTS patient, N1-3: negative as serum of ST patient).

**[Best Mode]**

The present invention provides a composition for improving sensitivity and specificity of nucleic acid detection, comprising protein binding to a target nucleic acid sequence; or a complex of the protein and a gRNA (guide RNA) binding thereto, as an active ingredient.

In particular, the protein binding to the target nucleic acid sequence may be a zinc finger protein or a transcription activator-like effector protein, but it is not limited thereto.

Specifically, a complex of the protein and a gRNA (guide RNA) binding thereto may be a complex of dCas9 (dead Cas, nuclease-inactive Cas9) protein and a gRNA binding thereto or a complex of dCpf1 (dead Cpf1, nuclease-inactive Cpf1) protein and a gRNA binding thereto, but it is not limited thereto.

Most preferably, the present invention can comprise a complex of dCas9 (dead cas, nuclease-inactive Cas9) protein and a gRNA binding thereto as an active ingredient and the sensitivity and the specificity of nucleic acid detection are greatly improved.

Accordingly, the present invention provides a composition for improving sensitivity and specificity of nucleic acid detection, comprising dCas9 (dead Cas, nuclease-inactive Cas9) protein and gRNA (guide RNA) binding to a target nucleic acid sequence, as an active ingredient. Preferably, the dCas9 protein may be represented by the amino acid sequence of SEQ ID NO: 1, but it is not limited thereto.

The target may be any one of causative organism of an infectious disease selected from the group consisting of Orientia tsutsugamushi (OT), Bunyavirus, Mycobacterium tuberculosis, Mers virus and respiratory virus, but it is limited thereto.

In particular, the composition may further comprise a nucleic acid polymerase, a primer capable of amplifying the target nucleic acid and a buffer solution.

In detail, the nucleic acid is not particularly limited, but may be any DNA or RNA, and may be chromosomal DNA, mitochondrial DNA, mRNA, rRNA, tRNA, miRNA, cfDNA, cfRNA, ctDNA and the like, which are present in the cell.

The 'dCas9' of the present invention is a variant of Cas9 in which the 10th aspartic acid is changed to alanine and the 840th histidine is changed to alanine among amino acids thereof, thereby suppressing nuclease activity. According to previous report, Cas9 WT and dCas9 were treated with gRNA and DNA together and then subjected to electrophoresis, resulting that the Cas9 WT cuts the target DNA thereby obtaining the cleavage fragment but dCas9 does not obtain the cleavage fragment. This shows that nuclease activity of dCas9 is inhibited (Conformational control of DNA target cleavage by CRISPR-Cas9, Nature 527,110-113).

Also, the present invention provides a kit for improving sensitivity and specificity of nucleic acid detection comprising dCas9 (dead cas, nuclease-inactive Cas9) protein and gRNA (guide RNA) binding to a target nucleic acid sequence as an active ingredient.

In addition, the present invention provides a method of improving sensitivity and specificity of nucleic acid detection, comprising amplifying a target nucleic acid by adding a dCas9 protein and a gRNA (guide RNA) binding to a target nucleic acid sequence to a sample containing the target nucleic acid; and detecting an amplified target nucleic acid amplification product. Preferably, the dCas9 protein may be represented by an amino acid sequence of SEQ ID NO: 1, but it is not limited thereto.

In detail, the step of amplifying the target nucleic acid is not particularly limited as long as it can amplify the target nucleic acid, however, the target nucleic acid may be amplified by using PCR, real-time PCR (RT-PCR), reverse transcriptase PCR, isothermal nucleic acid amplification and Silicon Microring Resonator (SMR), etc.

Specifically, the nucleic acid is not particularly limited, but may be any DNA or RNA, and may be chromosomal DNA, mitochondrial DNA, mRNA, rRNA, tRNA, miRNA and the like, which are present in the cell.

Meanwhile, the amplified product can be detected by methods known in the art, for example, gel electrophoresis, ELGA (enzyme-linked gel assay), ECL (electrochromiluminescent), fluorescent material, radioactive isotopes and the like can be used.

Examples of the fluorescent material include a rhodamine including rhodamine, TAMRA, etc.; fluorescein including fluorescein, FITC (fluorescein isothiocyanate) and FAM (fluorecein amidite), etc.; bodipy (boron-dipyrromethene); alexa fluor; and cyanine including Cy3, Cy5, Cy7, indocyanine green, but it is not limited thereto.

Examples of the radioisotope include H-3, C-14, P-32, S-35, Cl-36, Cr-51, Co-57, Co-58, Cu-64, Fe-59, Y-90, I-124, I-125, Re-186, I-131, Tc-99m, Mo-99, P-32, CR-51, Ca-45, Ca-68, etc. but particularly it is not limited thereto.

Hereinafter, the present invention will be described in detail with reference to the following examples. It should be noted, however, that the following examples are illustrative of the present invention and are not intended to limit the scope of the present invention. The examples of the present invention are provided to more fully describe the present invention to those skilled in the art.

**<Experimental Example>**

The following experimental examples are intended to provide experimental examples that are commonly applied to the respective examples according to the present invention.

**1. Protein purification**

For recombinant dCas9 RNP (ribonucleoprotein) purification, T7 Express BL21 (DE3) E. coli cells were transformed with the pET28a-His6-dCas9 plasmid. E. coli was cultured in Luria-Bertani (LB) broth at 30 °C until the OD600 reached 0.5-0.7 and then cultured in 0.2 mM isopropyl β-d-l-thiogaiactopyranoside (IPTG) to induce protein expression. Cell pellets were obtained by centrifuging at 5,000 g and eluting by sonication in elution buffer [50 mM NaH$_2$PO$_4$, 300 mM NaCl, 10 mM imidazole (pH 8.0), 1 mM PMSF, 1 mM DTT, 1 mg/mL lysozyme]. The aqueous eluent was obtained by centrifugation at 8,000 g and reacted with Ni-NTA agarose beads for 1-2 hours (Qiagen). Protein-bound Ni-NTA agarose beads were washed with a washing solution [50 mM NaH$_2$PO$_4$, 300 mM NaCl, 20 mM imidazole (pH 8.0)] and the dCas9 protein was eluted with imidazole containing buffer [50 mM NaH$_2$PO$_4$, 300 mM NaCl, 250 mM Imidazole (pH 8.0)]. Protein buffer eluted with 100 K Amicon centrifugal filter (Millipore) was ion-exchanged, concentrated and analyzed with 4-12% Bis-Tris gel (ThermoFisher).

**2. *In vitro* cleavage analysis**

The PCR product (400 ng) containing each of the Orientia Tsutsugamushi (OT) and severe fever with thrombocytopenia syndrome (SFTS) DNA sequences was mixed with 5.9 μl of rehydration buffer and 0.5 μl of 280 mM magnesium acetate (MgAc)) solution [provided by TwistAmp Basic RT kit]. 10 μl of the PCR product mixed with the buffer was reacted with 1 μg of Cas9 protein and 750 ng of sgRNA at 37 °C for 1 hour. The same PCR products as the positive control of cleavage assays were cleaved with 1x Buffer 3.1 (New England BioLabs) conditions. RNase A (4 μg) was added to remove sgRNA and the final product was analyzed by agarose gel electrophoresis.

**3. *In vitro* binding assay**

The dsDNA template was prepared by annealing the 5'-biotinylated target DNA strand and the non-biotinylated non-target DNA strand at 1:1.5 molar ratio. Here, the respective sequences are as follows:

1) 5'-biotinylated target DNA strand of Orientia Tsutsugamushi
OT_1_F_biotin: TATAAAGATCTTGTTA AATTGCAGCGTCATGCAGGAATTAGGAAAGC (SEQ ID NO: 2)
2) non-biotinylated non-target DNA strand of Orientia Tsutsugamushi
OT_1_R: GCTTTCCTAATTCCTGCATGACG CTGCAATTTAACAAGATCTTTATA (SEQ ID NO: 3)
3) 5'-biotinylated target DNA strand of SFTS
SFTS_F_biotin: AAAAATTAGCTGCCCAACAAGAAGAA GATGCAAAGAATCAAGGTGAA (SEQ ID NO: 4)
4) non-biotinylated non-target DNA strand of SFTS
SFTS_R: TTCACCTTGATTCTTTGCATCTTCTTCTTGTTGG GCAGCTAATTTTT (SEQ ID NO: 5)

10 nM dsDNA was reacted with 300 nM dCas9 and 1 μM sgRNA under cleavage buffer conditions. After reacting at 37 °C for 20 minutes, the cells were treated with 10% TBE gel using 0.5x TBE buffer supplemented with 5 mM MgCl$_2$. The in vitro binding state was analyzed by electrophoretic mobility analysis (EMSA) according to the manufacturer's instructions using chemiluminescent nucleic acid detection module kit (ThermoFisher) and Biodyne B nylon membrane (ThermoFisher).

**4. Construction of Silicon Microring Resonator (SMR)**

SMR and RPA (Recombinase Polymerase Amplification) were constructed and operated according to a conventionally known method so as to use SMR biosensor as a detection system for detecting target nucleic acid.
First, an SMR biosensor was treated with oxygen plasma cleaning (electric power: 100W, O$_2$: 80 sccm) for 1 minute and was soaked in 2% 3-aminopropyltriethoxysilane (APTES) dissolved in 95% ethanol at room temperature for 2 hours. The SMR biosensor was then cured at 120 °C for 15 minutes. Thereafter, the SMR biosensor was reacted with 2.5% glutaraldehyde (GAD) dissolved in deionized water containing 10 mM sodium cyanoborohydride for 1 hour at room temperature, rinsed with deionized water and dried under high purity nitrogen gas. Next, in order to immobilize the target primer on the SMR biosensor, the biosensor was reacted with the target primer dissolved in PBS (1 mM) containing 20 mM sodium cyanoborohydride solution for 16 hours at room temperature and rinsed with PBS to remove unbound target primers. At this time, an amine group was introduced at 5'-position of the target primer. Primers for detection of ST and SFTS were designed using the SFTS-S fragment and the ST-56-kDa type-specific gene, respectively (Table 1).

[Table 1]

| Analysis method | name | Sequence (5'-3') | SEQ ID NO |
|---|---|---|---|
| RT-PCR | SFTS-F | CGAGAGAGCTGGCCTATGAA | SEQ ID NO: 6 |
| | SFTS-R | TTCCCTGATGCCTTGACGAT | SEQ ID NO: 7 |
| | ST-F | GCAGCAGCTGTTAGGCTTTT | SEQ ID NO: 8 |
| | ST-R | TTGCAGTCACCTTCACCTTG | SEQ ID NO: 9 |
| SMR biosensor | SFTS-F | GGAGGCCTACTCTCTGTGGCAAGATGCCTTCA | SEQ ID NO: 10 |
| | SFTS-R | GGCCTTCAGCCACTTTACCCGAACATCATTGG | SEQ ID NO: 11 |
| | ST-F | GCAGCAGCAGCTGTTAGGCTTTTAAATGGCAATG | SEQ ID NO: 12 |
| | ST-R | GCTGCTTGCAGTCACCTTCACCTTGATTCTTTG | SEQ ID NO: 13 |

**5. SMR biosensor alone and CRISPR-mediated biosensor operation**

RPA and RT-RPA solutions were prepared respectively to amplify and detect the target nucleic acid using SMR biosensor alone. To prepare the RPA and RT-RPA solutions, 29.5 μl of rehydration buffer, 15 μl of RNase inhibitor and water and 2.5 μl of each 10 μM primer were mixed. The reaction mixture was then added to the lyophilized enzyme and 2.5 μl of a 280 mM magnesium acetate (MgAc) solution was dispensed into the cap of each tube, was added. Unidirectional shaking blending was performed for homogeneous dispensing. After mixing, 50 μl of the reaction buffer was divided into 10 μl droplets five times. To initiate the reaction for detection, 5 μl of the nucleic acid extracted from the patient's serum and 3 μl of dCas9 RNP (300 ng dCas9 and 225 ng gRNA) were added to each 10 μl reaction liquid droplets, and the biosensor was placed on a thermo electric cooler (TEC, Alpha Omega Instruments) equipped with a controller and a constant DC voltage was applied and maintained at a constant temperature (38 °C for DNA and 43 °C for RNA). The resonance spectrum of the biosensor was measured immediately, and the reference was used to obtain the baseline. The wavelength shift was monitored every 5 minutes up to 30 minutes and the amplification of the target nucleic acid was monitored in an unlabeled and real time manner. The relative resonance wavelength shift was calculated by the following equation.

$$\Delta\Delta pm = (\text{target wavelength value, pm}) - (\text{non-target wavelength value, pm})$$

**6. Extraction and preparation of nucleic acid samples**

Viral RNA was extracted from SFTS samples using QIAamp Viral RNA Kit (Qiagen Inc., Chatsworth, CA, USA) and genomic DNA was extracted from ST samples using QIAamp DNA mini kit (Qiagen). To prepare a SFTS viral RNA transcriptome control, an RNA fragment containing the target region was amplified with a primer containing the T7 promoter sequence on the antisense strand. Amplification products were transcribed in vitro using the MEGAscript T7 Transcription Kit (Ambion Life Technologies, Carlsbad, CA, USA). The synthetic RNA transcriptomes were purified using MEGAclear Kit (Ambion) and quantified with a Nanodrop spectrophotometer (Thermo Scientific, Waltham, MA, USA).

To prepare the ST bacterial DNA control, a DNA fragment containing the target region was amplified by PCR. The amplified DNA fragments were quantified with a Nanodrop spectrophotometer (Thermo Scientific, Waltham, MA, USA).

**7. Real-time PCR and real-time RT-PCR analysis**

The target DNA used as a template was obtained from clinical samples and real-time PCR and real-time RT-PCR analysis were performed using the primers shown in Table 1. Real-time PCR was performed with a denaturation step at 95 °C for 15 minutes, 45 cycles of 30 seconds at 95 °C, 30 seconds at 55 °C, and 30 seconds at 72 °C, and a final extension step at 72 °C for 10 minutes. The target DNA (5 $\mu$l) was amplified in a total 20 $\mu$l of the reactant [2x brilliant SYBR green RT-qPCR master mix and 25 pmol of each primer].

And real-time RT-PCR analysis was performed by modifying the AriaMx (Aligent) Instrument protocol as follows. Namely, the target RNA (5 $\mu$l) was amplified in a total 20 $\mu$l of the reactant [2x brilliant SYBR green RT-qPCR master mix and 25 pmol of each primer]. The initial cDNA synthesis step was carried out for 20 minutes at 50 °C, 15 cycles of 15 minutes at 95 °C, 15 seconds at 95 °C, 20 seconds at 55 °C, and 20 seconds at 72 °C and a cooling step at 40 °C for 30 seconds. SYBR Green signal of amplification product was obtained using AriaMx Real-Time PCR System (Agilent).

**8. Clinical Sample Preparation**

ST and SFTS serum samples were collected from patients at the Asan Medical Center. For SFTS, viral RNA was detected by real-time RT-PCR in serum using DiaStar 2X OneStep RT-PCR Pre-Mix kit (SolGent, Daejeon, South Korea). ST diagnosis was determined by confirming the single positive result of Immunofluorescence analysis (IFA; SD Bioline Tsutsugamushi Assay; Standard Diagnostics, Yongin, South Korea) or $\geq$ 1: 640 or 4-fold increase of IFA titre in continuous samples. These protocols were approved by IRB (Institutional Review Board) of the Asan Medical Center and proceeded with consent from all participants.

**<Example 1> Sensitivity and specificity analysis of CRISPR-mediated biosensor**

As shown in FIG. 1, pathogenic nucleic acid could be detected by using the combination of CRISPR/dCas9 and SMR biosensor from clinical samples in real time without any labeling. In order to detect these pathogenic nucleic acids, gRNAs targeting Orientia tsutsugamushi (OT), a causative organism of the scrub typhus (ST), and tuberculosis virus, which causes the severe fever with thrombocytopenia syndrome (SFTS) were prepared, respectively (FIG. 2 and FIG. 3).

Through in vitro cleavage analysis and electrophoretic mobility analysis (EMSA), in RPA buffer, it was observed that the cleavage of the target DNA was induced in Cas9 RNP and the target DNA was bound to dCas9 RNP (FIG. 4 and FIG. 5).

Also, in order to investigate whether dCas9 RNP improves detection sensitivity in SMR biosensors, DNA fragments was amplified from ST samples and signal enhancement was observed in dCas9 RNP-treated ST, which was compared with ST alone and ST treated with Cas9 RNP and as shown in FIG. 6, the detection sensitivity of ST treated with dCas9 RNP was improved. This sensitivity enhancement was due to the specific binding of dCas9 RNP to the target fragment on the SMR biosensor, the amplification efficiency is doubled than the conventional one and the difference between the non-target and the target was increased at least four times. In particular, the non-specific amplification of the non-target was prevented and the sensitivity and specificity were improved remarkably (FIG. 7).

In addition, as shown in FIG. 8, dCas9 RNP was treated by concentration to improve detection sensitivity of pathogenic nucleic acid, and 3x dCas9 RNP (300 ng of dCas9 + 225 ng of gRNA) showed the highest detection efficiency of pathogenic nucleic acid. [1x dCas9 RNP (100 ng dCas9 + 75 ng gRNA), 3x dCas9 RNP (300 ng dCas9 + 225 ng gRNA), 5x dCas9 RNP (500 ng dCas9 + 375 ng gRNA)]

The detection limit of the CRISPR-mediated biosensor according to the present invention was confirmed to be detected as ST (0.54 aM) and SFTS (0.63 aM) within 30 minutes using dCas9 RNP as shown in FIG. 9A and FIG. 9B, respectively. This detection limit was confirmed to be superior to the detection limit of the SMR biosensor ($\sim$ 10 copies) (FIG. 9a, FIG. 9b) and real time PCR ($\sim$ 100 copies) (FIG. 10).

Therefore, using the CRISPR-mediated biosensor according to the present invention, pathogenic nucleic acids such as ST and SFTS could be detected more sensitively than SMR biosensors alone or real-time PCR methods.

**<Example 2> Clinical sample analysis using CRISPR-mediated biosensor**

The present inventors have investigated whether the CRISPR-mediated biosensor according to the present invention is useful for clinical application in a novel infectious disease requiring promptness, high sensitivity specificity. Because clinical symptoms of SFTS and ST substantially overlaps, molecular diagnostic tests to distinguish them early are important and are essential to provide ST-specific antimicrobial treatments and appropriate preventative measures for SFTS.

Thus, in the present invention, clinical samples were selected from a total six patients consisting of three patients with ST and three patients with SFTS.

As a result of analyzing the diagnostic sample of ST with a CRISPR-mediated biosensor having an ST primer, an improved signal was observed only in the ST sample, not in the SFTS sample, as shown in FIG. 11A. In addition, as a result of analyzing these samples by a CRISPR-mediated biosensor having a SFTS primer to diagnose SFTS, an improved signal was observed only in the SFTS sample, as shown in FIG. 11B.

Therefore, the CRISPR-mediated biosensor according to the present invention can quickly and sensitively and specifically clearly distinguish ST and SFTS from clinical samples.

**Claims**

1. A composition for improving sensitivity and specificity of nucleic acid detection, comprising:
   protein binding to a target nucleic acid sequence; or a complex of the protein and a gRNA (guide RNA) binding thereto, as an active ingredient.

2. A composition for improving sensitivity and specificity of nucleic acid detection, comprising:
   dCas9 (dead Cas, nuclease-inactive Cas9) protein and gRNA (guide RNA) binding to a target nucleic acid sequence, as an active ingredient.

**3.** The composition for improving sensitivity and specificity of nucleic acid detection of claim 2, wherein the dCas9 protein is represented by an amino acid sequence of SEQ ID NO: 1.

**4.** The composition for improving sensitivity and specificity of nucleic acid detection of claim 2, wherein the target is a causative organism of an infectious disease selected from the group consisting of Orientia tsutsugamushi (OT), Bunyavirus, Mycobacterium tuberculosis, Mers virus and respiratory virus.

**5.** The composition for improving sensitivity and specificity of nucleic acid detection of claim 2, wherein the composition further comprises a nucleic acid polymerase, a primer capable of amplifying a target nucleic acid and a buffer solution.

**6.** The composition for improving sensitivity and specificity of nucleic acid detection of claim 2, wherein the nucleic acid is DNA or RNA.

**7.** A kit for improving sensitivity and specificity of nucleic acid detection comprising the composition of any one of claims 1 to 6.

**8.** A method of improving sensitivity and specificity of nucleic acid detection, comprising:

amplifying a target nucleic acid by adding a dCas9 protein and a gRNA (guide RNA) binding to a target nucleic acid sequence to a sample containing the target nucleic acid; and
detecting an amplified target nucleic acid amplification product.

**9.** The method of improving sensitivity and specificity of nucleic acid detection of claim 8, wherein the dCas9 protein is represented by an amino acid sequence of SEQ ID NO: 1.

**10.** The method of improving sensitivity and specificity of nucleic acid detection of claim 8, wherein the target nucleic acid is amplified by using at least one selected from the group consisting of PCR, real-time PCR (RT-PCR), reverse transcriptase PCR, isothermal nucleic acid amplification and Silicon Microring Resonator (SMR).

**11.** The method of improving sensitivity and specificity of nucleic acid detection of claim 8, wherein the nucleic acid is DNA or RNA.

**FIG. 1**

**FIG. 2**

**FIG. 3**

*Bunyavirus* (BUN, ssRNA target of SFTS)

————————CCGCGCAUCUUCACAUUGAUAGU————

Reverse Transcription

CCGCGCATCTTCACATTGATAGT
GGCGCGTAGAAGTGTAACTATCA

**FIG. 4**

**FIG. 5**

**FIG. 6**

**FIG. 7**

**FIG. 8**

**FIG. 9a**

**FIG. 9b**

**FIG. 10**

**FIG. 11a**

FIG. 11b

## INTERNATIONAL SEARCH REPORT

| | International application No. |
|---|---|
| | **PCT/KR2017/009765** |

**A. CLASSIFICATION OF SUBJECT MATTER**

*C12Q 1/68(2006.01)i*

According to International Patent Classification (IPC) or to both national classification and IPC

**B. FIELDS SEARCHED**

Minimum documentation searched (classification system followed by classification symbols)
C12Q 1/68; C07K 19/00; C12N 5/00; C12N 9/96; C07K 14/195

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: gRNA, dCas9, orientia tsutsugamushi, bunyavirus, tubercular bacillus, MERSC, PCR, nucleic acid detection

**C. DOCUMENTS CONSIDERED TO BE RELEVANT**

| Category* | Citation of document, with indication, where appropriate, of the relevant passages | Relevant to claim No. |
|---|---|---|
| X | KR 10-2015-0138075 A (TOOLGEN INCORPORATED et al.) 09 December 2015 See abstract; paragraphs [0009]-[0010], [0070], [0142]; claims 1-9, 41-47. | 1-2,4-8,10-11 |
| Y | | 3,9 |
| Y | NCBI, GenBank accession no. BAV54124.1 (23 August 2016) See DEFINITION; ORIGIN. | 3,9 |
| A | US 2016-0010076 A1 (THE GENERAL HOSPITAL CORPORATION) 14 January 2016 See the entire document. | 1-11 |
| A | KR 10-2015-0126946 A (CARIBOU BIOSCIENCES, INC.) 13 November 2015 See the entire document. | 1-11 |
| A | MORITA, Sumiyo et al., "Targeted DNA Demethylation in Vivo Using dCas9-peptide Repeat and scFv-TET1 Catalytic Domain Fusions", Nature Biotechnology, Published online 29 August 2016, vol. 34, no. 10, pages 1060-1065 See the entire document. | 1-11 |

☐ Further documents are listed in the continuation of Box C. ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 DECEMBER 2017 (21.12.2017) | **21 DECEMBER 2017 (21.12.2017)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. +82-42-481-8578 | Telephone No. |

Form PCT/ISA/210 (second sheet) (January 2015)

INTERNATIONAL SEARCH REPORT
Information on patent family members

International application No.

**PCT/KR2017/009765**

| Patent document cited in search report | Publication date | Patent family member | Publication date |
| --- | --- | --- | --- |
| KR 10-2015-0138075 A | 09/12/2015 | CN 106687601 A | 17/05/2017 |
| | | EP 3150718 A1 | 05/04/2017 |
| | | JP 2017-516500 A | 22/06/2017 |
| | | KR 10-2015-0138074 A | 09/12/2015 |
| | | US 2017-0191123 A1 | 06/07/2017 |
| | | WO 2015-183025 A1 | 03/12/2015 |
| | | WO 2015-183026 A1 | 03/12/2015 |
| US 2016-0010076 A1 | 14/01/2016 | AU 2014-227653 A1 | 01/10/2015 |
| | | AU 2014-227653 A2 | 12/11/2015 |
| | | AU 2014-227653 B2 | 20/04/2017 |
| | | AU 2014-228981 A1 | 01/10/2015 |
| | | AU 2014-239665 A1 | 01/10/2015 |
| | | AU 2014-370416 A1 | 02/07/2015 |
| | | AU 2017-204909 A1 | 31/08/2017 |
| | | CA 2906553 A1 | 25/09/2014 |
| | | CA 2906724 A1 | 18/09/2014 |
| | | CA 2907198 A1 | 18/09/2014 |
| | | CA 2935032 A1 | 02/07/2015 |
| | | CN 105247066 A | 13/01/2016 |
| | | CN 105408483 A | 16/03/2016 |
| | | CN 105408497 A | 16/03/2016 |
| | | CN 106103706 A | 09/11/2016 |
| | | EP 2970208 A2 | 20/01/2016 |
| | | EP 2970986 A2 | 20/01/2016 |
| | | EP 2971041 A1 | 20/01/2016 |
| | | EP 2971125 A2 | 20/01/2016 |
| | | EP 3090044 A1 | 09/11/2016 |
| | | JP 2016-512264 A | 25/04/2016 |
| | | JP 2016-512691 A | 09/05/2016 |
| | | JP 2016-517276 A | 16/06/2016 |
| | | KR 10-2015-0131250 A | 24/11/2015 |
| | | KR 10-2015-0131251 A | 24/11/2015 |
| | | KR 10-2015-0132395 A | 25/11/2015 |
| | | KR 10-2016-0102056 A | 26/08/2016 |
| | | US 2014-0295556 A1 | 02/10/2014 |
| | | US 2014-0295557 A1 | 02/10/2014 |
| | | US 2014-0377868 A1 | 25/12/2014 |
| | | US 2016-0024523 A1 | 28/01/2016 |
| | | US 2016-0024524 A1 | 28/01/2016 |
| | | US 2016-0153003 A1 | 02/06/2016 |
| | | US 2016-0319281 A1 | 03/11/2016 |
| | | US 2017-0152508 A1 | 01/06/2017 |
| | | US 9567603 B2 | 14/02/2017 |
| | | US 9567604 B2 | 14/02/2017 |
| | | WO 2014-144288 A1 | 18/09/2014 |
| | | WO 2014-144592 A2 | 18/09/2014 |
| | | WO 2014-144592 A3 | 31/12/2014 |
| | | WO 2014-144761 A2 | 18/09/2014 |

Form PCT/ISA/210 (patent family annex) (January 2015)

**INTERNATIONAL SEARCH REPORT**
Information on patent family members

| | | International application No. |
|---|---|---|
| | | **PCT/KR2017/009765** |

| Patent document cited in search report | Publication date | Patent family member | Publication date |
|---|---|---|---|
| | | WO 2014-144761 A3 | 29/10/2015 |
| | | WO 2014-152432 A2 | 25/09/2014 |
| | | WO 2014-152432 A3 | 29/10/2015 |
| | | WO 2014-204578 A1 | 24/12/2014 |
| | | WO 2015-099850 A1 | 02/07/2015 |
| KR 10-2015-0126946 A | 13/11/2015 | AU 2014-235794 A1 | 22/10/2015 |
| | | AU 2015-101418 A4 | 12/11/2015 |
| | | AU 2015-101418 A6 | 04/02/2016 |
| | | AU 2017-200138 A1 | 02/02/2017 |
| | | CA 2905432 A1 | 25/09/2014 |
| | | CN 105980575 A | 28/09/2016 |
| | | EP 2971167 A1 | 20/01/2016 |
| | | JP 2016-519652 A | 07/07/2016 |
| | | KR 10-2017-0108172 A | 26/09/2017 |
| | | US 2014-0315985 A1 | 23/10/2014 |
| | | US 2016-0024568 A1 | 28/01/2016 |
| | | US 2016-0046949 A1 | 18/02/2016 |
| | | US 2016-0046962 A1 | 18/02/2016 |
| | | US 2016-0046963 A1 | 18/02/2016 |
| | | US 2016-0046978 A1 | 18/02/2016 |
| | | US 2016-0068887 A1 | 10/03/2016 |
| | | US 2016-0076020 A1 | 17/03/2016 |
| | | US 2016-0108470 A1 | 21/04/2016 |
| | | US 2016-0251640 A1 | 01/09/2016 |
| | | US 2016-0312280 A1 | 27/10/2016 |
| | | US 2016-0319349 A1 | 03/11/2016 |
| | | US 2017-0051276 A1 | 23/02/2017 |
| | | US 9260752 B1 | 16/02/2016 |
| | | US 9410198 B2 | 09/08/2016 |
| | | US 9725714 B2 | 08/08/2017 |
| | | US 9803194 B2 | 31/10/2017 |
| | | WO 2014-150624 A1 | 25/09/2014 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• *Nature,* vol. 527, 110-113

<110>    University of Ulsan Foundation For Industry Cooperation
         THE ASAN FOUNDATION

<120>    Composition and method for improving sensitivity and specificity
         on detection of nucleic acids with using dCas9 protein and gRNA
         for binding to target nucleic acid sequence

<130>    ADP-2017-0063PCT

<150>    KR 10-2016-0115024
<151>    2016-09-07

<150>    KR 10-2017-0112474
<151>    2017-09-04


<160>    13

<170>    KopatentIn 2.0

<210>    1
<211>    1389
<212>    PRT
<213>    Artificial Sequence

<220>
<223>    nuclease-inactive Cas9


<400>    1
Met Asp Lys Lys Tyr Ser Ile Gly Leu Ala Ile Gly Thr Asn Ser Val
  1               5                  10                  15

Gly Trp Ala Val Ile Thr Asp Glu Tyr Lys Val Pro Ser Lys Lys Phe
             20                  25                  30

Lys Val Leu Gly Asn Thr Asp Arg His Ser Ile Lys Lys Asn Leu Ile
         35                  40                  45

Gly Ala Leu Leu Phe Asp Ser Gly Glu Thr Ala Glu Ala Thr Arg Leu
     50                  55                  60

Lys Arg Thr Ala Arg Arg Arg Tyr Thr Arg Arg Lys Asn Arg Ile Cys
 65                  70                  75                  80

Tyr Leu Gln Glu Ile Phe Ser Asn Glu Met Ala Lys Val Asp Asp Ser
             85                  90                  95

Phe Phe His Arg Leu Glu Glu Ser Phe Leu Val Glu Glu Asp Lys Lys
             100                 105                 110

His Glu Arg His Pro Ile Phe Gly Asn Ile Val Asp Glu Val Ala Tyr
         115                 120                 125

His Glu Lys Tyr Pro Thr Ile Tyr His Leu Arg Lys Lys Leu Val Asp
     130                 135                 140

Ser Thr Asp Lys Ala Asp Leu Arg Leu Ile Tyr Leu Ala Leu Ala His
145                 150                 155                 160

Met Ile Lys Phe Arg Gly His Phe Leu Ile Glu Gly Asp Leu Asn Pro
                 165                 170                 175

Asp Asn Ser Asp Val Asp Lys Leu Phe Ile Gln Leu Val Gln Thr Tyr
                180                 185                 190

Asn Gln Leu Phe Glu Glu Asn Pro Ile Asn Ala Ser Gly Val Asp Ala
        195                 200                 205

Lys Ala Ile Leu Ser Ala Arg Leu Ser Lys Ser Arg Arg Leu Glu Asn
    210                 215                 220

Leu Ile Ala Gln Leu Pro Gly Glu Lys Lys Asn Gly Leu Phe Gly Asn
225                 230                 235                 240

Leu Ile Ala Leu Ser Leu Gly Leu Thr Pro Asn Phe Lys Ser Asn Phe
                245                 250                 255

Asp Leu Ala Glu Asp Ala Lys Leu Gln Leu Ser Lys Asp Thr Tyr Asp
            260                 265                 270

Asp Asp Leu Asp Asn Leu Leu Ala Gln Ile Gly Asp Gln Tyr Ala Asp
        275                 280                 285

Leu Phe Leu Ala Ala Lys Asn Leu Ser Asp Ala Ile Leu Leu Ser Asp
        290                 295                 300

Ile Leu Arg Val Asn Thr Glu Ile Thr Lys Ala Pro Leu Ser Ala Ser
305                 310                 315                 320

Met Ile Lys Arg Tyr Asp Glu His His Gln Asp Leu Thr Leu Leu Lys
                325                 330                 335

Ala Leu Val Arg Gln Gln Leu Pro Glu Lys Tyr Lys Glu Ile Phe Phe
            340                 345                 350

Asp Gln Ser Lys Asn Gly Tyr Ala Gly Tyr Ile Asp Gly Gly Ala Ser
        355                 360                 365

Gln Glu Glu Phe Tyr Lys Phe Ile Lys Pro Ile Leu Glu Lys Met Asp
    370                 375                 380

Gly Thr Glu Glu Leu Leu Val Lys Leu Asn Arg Glu Asp Leu Leu Arg
385                 390                 395                 400

Lys Gln Arg Thr Phe Asp Asn Gly Ser Ile Pro His Gln Ile His Leu
            405                 410                 415

Gly Glu Leu His Ala Ile Leu Arg Arg Gln Glu Asp Phe Tyr Pro Phe
            420                 425                 430

Leu Lys Asp Asn Arg Glu Lys Ile Glu Lys Ile Leu Thr Phe Arg Ile
        435                 440                 445

Pro Tyr Tyr Val Gly Pro Leu Ala Arg Gly Asn Ser Arg Phe Ala Trp
    450                 455                 460

Met Thr Arg Lys Ser Glu Glu Thr Ile Thr Pro Trp Asn Phe Glu Glu
465                 470                 475                 480

Val Val Asp Lys Gly Ala Ser Ala Gln Ser Phe Ile Glu Arg Met Thr
            485                 490                 495

Asn Phe Asp Lys Asn Leu Pro Asn Glu Lys Val Leu Pro Lys His Ser
        500                 505                 510

```
Leu Leu Tyr Glu Tyr Phe Thr Val Tyr Asn Glu Leu Thr Lys Val Lys
        515             520             525

Tyr Val Thr Glu Gly Met Arg Lys Pro Ala Phe Leu Ser Gly Glu Gln
    530             535             540

Lys Lys Ala Ile Val Asp Leu Leu Phe Lys Thr Asn Arg Lys Val Thr
545             550             555             560

Val Lys Gln Leu Lys Glu Asp Tyr Phe Lys Lys Ile Glu Cys Phe Asp
            565             570             575

Ser Val Glu Ile Ser Gly Val Glu Asp Arg Phe Asn Ala Ser Leu Gly
        580             585             590

Thr Tyr His Asp Leu Leu Lys Ile Ile Lys Asp Lys Asp Phe Leu Asp
    595             600             605

Asn Glu Glu Asn Glu Asp Ile Leu Glu Asp Ile Val Leu Thr Leu Thr
610             615             620

Leu Phe Glu Asp Arg Glu Met Ile Glu Glu Arg Leu Lys Thr Tyr Ala
625             630             635             640

His Leu Phe Asp Asp Lys Val Met Lys Gln Leu Lys Arg Arg Arg Tyr
            645             650             655

Thr Gly Trp Gly Arg Leu Ser Arg Lys Leu Ile Asn Gly Ile Arg Asp
            660             665             670

Lys Gln Ser Gly Lys Thr Ile Leu Asp Phe Leu Lys Ser Asp Gly Phe
    675             680             685

Ala Asn Arg Asn Phe Met Gln Leu Ile His Asp Asp Ser Leu Thr Phe
    690             695             700

Lys Glu Asp Ile Gln Lys Ala Gln Val Ser Gly Gln Gly Asp Ser Leu
705             710             715             720

His Glu His Ile Ala Asn Leu Ala Gly Ser Pro Ala Ile Lys Lys Gly
            725             730             735

Ile Leu Gln Thr Val Lys Val Val Asp Glu Leu Val Lys Val Met Gly
            740             745             750

Arg His Lys Pro Glu Asn Ile Val Ile Glu Met Ala Arg Glu Asn Gln
    755             760             765

Thr Thr Gln Lys Gly Gln Lys Asn Ser Arg Glu Arg Met Lys Arg Ile
    770             775             780

Glu Glu Gly Ile Lys Glu Leu Gly Ser Gln Ile Leu Lys Glu His Pro
785             790             795             800

Val Glu Asn Thr Gln Leu Gln Asn Glu Lys Leu Tyr Leu Tyr Tyr Leu
            805             810             815

Gln Asn Gly Arg Asp Met Tyr Val Asp Gln Glu Leu Asp Ile Asn Arg
            820             825             830

Leu Ser Asp Tyr Asp Val Asp Ala Ile Val Pro Gln Ser Phe Leu Lys
            835             840             845
```

```
Asp Asp Ser Ile Asp Asn Lys Val Leu Thr Arg Ser Asp Lys Asn Arg
    850             855             860

Gly Lys Ser Asp Asn Val Pro Ser Glu Glu Val Val Lys Lys Met Lys
865             870             875             880

Asn Tyr Trp Arg Gln Leu Leu Asn Ala Lys Leu Ile Thr Gln Arg Lys
            885             890             895

Phe Asp Asn Leu Thr Lys Ala Glu Arg Gly Gly Leu Ser Glu Leu Asp
        900             905             910

Lys Ala Gly Phe Ile Lys Arg Gln Leu Val Glu Thr Arg Gln Ile Thr
    915             920             925

Lys His Val Ala Gln Ile Leu Asp Ser Arg Met Asn Thr Lys Tyr Asp
    930             935             940

Glu Asn Asp Lys Leu Ile Arg Glu Val Lys Val Ile Thr Leu Lys Ser
945             950             955             960

Lys Leu Val Ser Asp Phe Arg Lys Asp Phe Gln Phe Tyr Lys Val Arg
            965             970             975

Glu Ile Asn Asn Tyr His His Ala His Asp Ala Tyr Leu Asn Ala Val
            980             985             990

Val Gly Thr Ala Leu Ile Lys Lys Tyr Pro Lys Leu Glu Ser Glu Phe
    995             1000            1005

Val Tyr Gly Asp Tyr Lys Val Tyr Asp Val Arg Lys Met Ile Ala Lys
    1010            1015            1020

Ser Glu Gln Glu Ile Gly Lys Ala Thr Ala Lys Tyr Phe Phe Tyr Ser
1025            1030            1035            1040

Asn Ile Met Asn Phe Phe Lys Thr Glu Ile Thr Leu Ala Asn Gly Glu
            1045            1050            1055

Ile Arg Lys Arg Pro Leu Ile Glu Thr Asn Gly Glu Thr Gly Glu Ile
        1060            1065            1070

Val Trp Asp Lys Gly Arg Asp Phe Ala Thr Val Arg Lys Val Leu Ser
        1075            1080            1085

Met Pro Gln Val Asn Ile Val Lys Lys Thr Glu Val Gln Thr Gly Gly
    1090            1095            1100

Phe Ser Lys Glu Ser Ile Leu Pro Lys Arg Asn Ser Asp Lys Leu Ile
1105            1110            1115            1120

Ala Arg Lys Lys Asp Trp Asp Pro Lys Lys Tyr Gly Gly Phe Asp Ser
            1125            1130            1135

Pro Thr Val Ala Tyr Ser Val Leu Val Val Ala Lys Val Glu Lys Gly
        1140            1145            1150

Lys Ser Lys Lys Leu Lys Ser Val Lys Glu Leu Leu Gly Ile Thr Ile
        1155            1160            1165

Met Glu Arg Ser Ser Phe Glu Lys Asn Pro Ile Asp Phe Leu Glu Ala
    1170            1175            1180
```

12

```
Lys Gly Tyr Lys Glu Val Lys Lys Asp Leu Ile Ile Lys Leu Pro Lys
1185            1190            1195            1200

Tyr Ser Leu Phe Glu Leu Glu Asn Gly Arg Lys Arg Met Leu Ala Ser
                1205            1210            1215

Ala Gly Glu Leu Gln Lys Gly Asn Glu Leu Ala Leu Pro Ser Lys Tyr
        1220            1225            1230

Val Asn Phe Leu Tyr Leu Ala Ser His Tyr Glu Lys Leu Lys Gly Ser
        1235            1240            1245

Pro Glu Asp Asn Glu Gln Lys Gln Leu Phe Val Glu Gln His Lys His
    1250            1255            1260

Tyr Leu Asp Glu Ile Ile Glu Gln Ile Ser Glu Phe Ser Lys Arg Val
1265            1270            1275            1280

Ile Leu Ala Asp Ala Asn Leu Asp Lys Val Leu Ser Ala Tyr Asn Lys
            1285            1290            1295

His Arg Asp Lys Pro Ile Arg Glu Gln Ala Glu Asn Ile Ile His Leu
        1300            1305            1310

Phe Thr Leu Thr Asn Leu Gly Ala Pro Ala Ala Phe Lys Tyr Phe Asp
        1315            1320            1325

Thr Thr Ile Asp Arg Lys Arg Tyr Thr Ser Thr Lys Glu Val Leu Asp
    1330            1335            1340

Ala Thr Leu Ile His Gln Ser Ile Thr Gly Leu Tyr Glu Thr Arg Ile
1345            1350            1355            1360

Asp Leu Ser Gln Leu Gly Gly Asp Gly Gly Ser Gly Pro Pro Lys Lys
            1365            1370            1375

Lys Arg Lys Val Tyr Pro Tyr Asp Val Pro Asp Tyr Ala
            1380            1385


<210>    2
<211>    47
<212>    DNA
<213>    Orientia Tsutsugamushi

<400>    2
tataaagatc ttgttaaatt gcagcgtcat gcaggaatta ggaaagc                       47


<210>    3
<211>    47
<212>    DNA
<213>    Orientia Tsutsugamushi

<400>    3
gctttcctaa ttcctgcatg acgctgcaat ttaacaagat ctttata                       47


<210>    4
<211>    47
<212>    DNA
<213>    Bunyavirus
```

13

<400>     4
aaaaattagc tgcccaacaa gaagaagatg caaagaatca aggtgaa                          47


<210>     5
<211>     47
<212>     DNA
<213>     Bunyavirus

<400>     5
ttcaccttga ttctttgcat cttcttcttg ttgggcagct aattttt                          47


<210>     6
<211>     20
<212>     DNA
<213>     Artificial Sequence

<220>
<223>     primer


<400>     6
cgagagagct ggcctatgaa                                                        20


<210>     7
<211>     20
<212>     DNA
<213>     Artificial Sequence

<220>
<223>     primer


<400>     7
ttccctgatg ccttgacgat                                                        20


<210>     8
<211>     20
<212>     DNA
<213>     Artificial Sequence

<220>
<223>     primer


<400>     8
gcagcagctg ttaggctttt                                                        20


<210>     9
<211>     20
<212>     DNA
<213>     Artificial Sequence

<220>
<223>     primer


<400>     9
ttgcagtcac cttcaccttg                                                        20

```
<210>      10
<211>      32
<212>      DNA
<213>      Artificial Sequence

<220>
<223>      primer


<400>      10
ggaggcctac tctctgtggc aagatgcctt ca                                  32


<210>      11
<211>      32
<212>      DNA
<213>      Artificial Sequence

<220>
<223>      primer


<400>      11
ggccttcagc cactttaccc gaacatcatt gg                                  32


<210>      12
<211>      34
<212>      DNA
<213>      Artificial Sequence

<220>
<223>      primer


<400>      12
gcagcagcag ctgttaggct tttaaatggc aatg                                34


<210>      13
<211>      33
<212>      DNA
<213>      Artificial Sequence

<220>
<223>      primer


<400>      13
gctgcttgca gtcaccttca ccttgattct ttg                                 33
```